# EUROPÄISCHE PATENTSCHRIFT

(11) **EP 0 678 705 B1**
(45) Veröffentlichungstag und Bekanntmachung des Hinweises auf die Patenterteilung: **12.07.2000**
(21) Anmeldenummer: 95105863.5
(22) Anmeldetag: 19.04.1995
(51) Int. Cl.: F21V 9/12, G02B 5/24

(54) **Bestrahlungsvorrichtung**
Irradiation device
Dispositif d'irradiation

(30) Priorität: 21.04.1994 DE 9406682 U
(43) Veröffentlichungstag der Anmeldung: 25.10.1995
(73) Patentinhaber: MAXS AG, CH-6072 Sachseln (CH)
(72) Erfinder: Braun, Werner, CH-6062 Wilen (CH)
(74) Vertreter: Grünecker, Kinkeldey, Stockmair & Schwanhäusser Anwaltssozietät

(56) Entgegenhaltungen:
- DE-C- 4 042 259
- US-A- 2 783 682
- US-A- 3 572 907

## Beschreibung

Die Erfindung bezieht sich auf eine Bestrahlungsvorrichtung mit einer Strahlenquelle und einem im Strahlengang angeordneten Filter, der zwei transparente, im wesentlichen planparallel zueinander angeordnete Scheiben aufweist, die mit ihren umlaufenden Rändern in einem Rahmen aus gut wärmeleitenden Material gehalten sind, wobei die Scheiben und der Rahmen einen geschlossenen Hohlraum begrenzen, in dem ein das Strahlenspektrum selektiv beeinflussendes Medium vorgesehen ist.

Eine solche Bestrahlungsvorrichtung ist beispielsweise aus der EP-A-0311898 bekannt. Die in dieser Druckschrift beschriebene Bestrahlungsvorrichtung wird für die Wärmetherapie insbesondere des menschlichen Körpers verwendet. Der Sinn des bei dieser Vorrichtung verwendeten Filters besteht darin, daß das von der Strahlenquelle ausgesandte Strahlenspektrum in seiner Gesamtheit beschränkt für die Wärmetherapie geeignet ist. Aus diesem Grunde werden von dem Filter bestimmte Banden aus dem Strahlenspektrum herausgefiltert. Der bekannte Filter besteht aus einem mehrteiligen Rahmen, der aus einem äußeren, mit dem Gehäuse der Bestrahlungsvorrichtung verbundenen Rahmen und einem darin angeordneten Wechselrahmen, der beide Filterscheiben trägt, aufgebaut ist. Am Wechselrahmen sind zwei Preßringe angeordnet, die dafür sorgen, daß die beiden Filterscheiben jeweils in Richtung des Hohlraumes auf ihre Dichtungen gepreßt werden. Mindestens einer der Preßringe wird mit dem Wechselrahmen durch einen Bördelvorgang nicht lösbar verbunden.

Die Aufgabe der vorliegenden Erfindung besteht darin, eine Bestrahlungsvorrichtung der eingangs genannten Art so zu verbessern, daß der Filter einen einfacheren Aufbau aufweisen kann.

Diese Aufgabe wird erfindungsgemäß dadurch gelöst, daß der Rahmen im wesentlichen quer zur Filterachse in eine erste und eine zweite Rahmenhälfte derart geteilt ist, daß die eine Scheibe auf der dem Hohlraum zugewandten Seite an der ersten Rahmenhälfte angeordnet ist und die andere Scheibe auf der dem Hohlraum zugewandten Seite an der zweiten Rahmenhälfte angeordnet ist. Durch diese Zweiteilung sorgen die Rahmenhälften nunmehr selbst für eine genaue und stabile Anordnung der Filterscheiben, ohne daß zusätzliche Preßringe erforderlich wären. Des weiteren können die beiden Rahmenhälften durch lösbare Verbindungsmittel aneinander angeordnet werden, so daß auch nach einiger Betriebszeit der Zugang auf das im Hohlraum befindliche Medium, sowie die Filterscheiben gewährt ist.

Bevorzugterweise ist zumindest eine der Scheiben entsprechend in einer umlaufenden Stufe der ersten und zweiten Rahmenhälfte im wesentlichen paßgenau eingesetzt. Die Stufe stellt eine Anschlagschulter für die Scheibe bereit, so daß diese durch den im Hohlraum vorherrschenden Druck nicht aus ihrer Halterung herausgedrückt werden kann.

Die Dichtwirkung dieses Filters läßt sich insbesondere dadurch erhöhen, daß die Scheiben jeweils an ihrem Rand auf der dem Hohlraum abgewandten Seite an einer im wesentlichen umlaufenden Dichtung anliegen. Entgegen dem Stand der Technik, sorgt der Druck im Inneren des Hohlraums bei dieser Anordnung dafür, daß sich bei Erhöhung des Drucks auch die Dichtwirkung erhöht, da die Scheiben immer fester auf ihre Dichtungen gepreßt werden.

Da der Filter im Strahlengang der Strahlenquelle angeordnet ist, ist er zugleich außerordentlich hohen Temperaturen ausgesetzt. Um die Konvektion des sich im Hohlraum befindlichen Mediums zu verbessern, kann mindestens in einer der Rahmenhälften eine umlaufende mit dem Hohlraum in Verbindung stehende Ringkammer angeordnet sein, wodurch die Wärme über das Rahmengehäuse, durch die vergrößerte Berührungsfläche zwischen Medium und Rahmen, schneller abtransportiert werden kann. Dabei ist es insbesondere von Vorteil, wenn in beiden Rahmenhälften jeweils eine Ringkammerhälfte zum Bilden einer gemeinsamen großen Ringkammer angeordnet ist. Durch Zusammenfügen der beiden Rahmenhälften entsteht somit eine relativ große Ringkammer, in der ein sehr guter Wärmeaustausch zwischen Rahmen und Medium stattfinden kann.

Damit die Ringkammer besser nach außen abgedichtet ist, kann außerhalb der Ringkammer mindestens eine umlaufende Dichtung in einer Rahmenhälfte angeordnet sein, die an der anderen Rahmenhälfte anliegt.

Für das richtige Funktionieren des Filters ist es insbesondere wichtig, daß die beiden Scheiben im wesentlichen planparallel zueinander angeordnet sind und auch nicht durch Wärmeausdehnung des Mediums und dem daraus resultierenden höheren Druck aus ihrer Planparallelität gebracht werden. Hierzu kann in oder an einer der Ringkammern eine Druckausgleichsvorrichtung vorgesehen sein, die Druckschwankungen aufgrund des Erwärmens des im Hohlraum befindlichen Mediums ausgleicht, sowie eine eventuelle Ansammlung von Gasblasen aufnimmt. Die Druckausgleichsvorrichtung stellt somit sicher, daß eine Verformung der Scheiben durch den Innendruck in der Hohlkammer nicht erfolgen kann.

Als besonders vorteilhaft hat sich herausgestellt, wenn die Druckausgleichsvorrichtung aus einem mit einer Ringkammer in Verbindung stehenden Ausgleichsbehälter besteht, der eine Ausgleichskammer aufweist, die zumindest einseitig von einer flexiblen Membran begrenzt ist. Die flexible Membran weicht dann entsprechend bei Druckanstieg oder Druckabfall in die jeweilige Richtung aus. Bei einer weiteren Variante der Druckausgleichsvorrichtung kann in der Ausgleichskammer des Ausgleichsbehälters ein federbelasteter Kolben angeordnet sein, der entsprechend bei Druckschwankungen im Hohlraum ausgleichend ein- oder ausfedert.

Da die Temperatur der der Strahlenquelle abgewandten Scheibe des Filters im wesentlichen von dem im Hohlraum befindlichen Medium bestimmt wird, kann diese Scheibe auch aus Kunststoff, vorzugsweise Polycarbonat, bestehen. Die Materialkosten lassen sich somit stark herabsetzen.

Die der Strahlenquelle zugewandte Scheibe kann in bevorzugter Weise aus einem hitzebeständigen Material, vorzugsweise Mineralglas, bestehen. Wenn die Strahlenquelle nicht ganz so leistungsstark ist, kann auch Polysulfon verwendet werden, ansonsten wird eine unter der Bezeichnung "Robax" gehandelte Glaskeramik bevorzugt.

Die vom Rahmen aufgenommene Wärme läßt sich noch schneller nach außen abführen, wenn an seinem Außenumfang Kühlrippen angeordnet sind.

Eine einfache Befestigung der beiden Rahmenhälften aneinander kann dadurch erreicht werden, daß einige Kühlrippen verdickt ausgeführt sind und entsprechend bezüglich der ersten und zweiten Rahmenhälfte fluchtende Durchgangsbohrungen zur Aufnahme von Befestigungsmitteln aufweisen.

Im folgenden wird ein Ausführungsbeispiel der Erfindung anhand einer Zeichnung näher erläutert. Es zeigt:
- Fig. 1: einen Filter zum Einsatz in der erfindungsgemäßen Bestrahlungsvorrichtung im Vollschnitt,
- Fig. 2: einen vergrößerten Ausschnitt des Filters aus Fig. 1,
- Fig. 3: eine erste Rahmenhälfte mit Scheibe des Filters aus Fig. 1 in einer Draufsicht,
- Fig. 4: eine Schnittdarstellung der ersten Rahmenhälfte entlang der Linie IV-IV in Fig. 3 geschnitten,
- Fig. 5: eine zweite Rahmenhälfte des Filters aus Fig. 1 in einer Draufsicht,
- Fig. 6: die zweite Rahmenhälfte entlang der Linie VI-VI in Fig. 5 geschnitten,
- Fig. 7: eine zweite Variante einer Druckausgleichsvorrichtung in vergrößerter Schnittdarstellung und
- Fig. 8: eine dritte Variante einer Druckausgleichsvorrichtung in vergrößerter Schnittdarstellung.

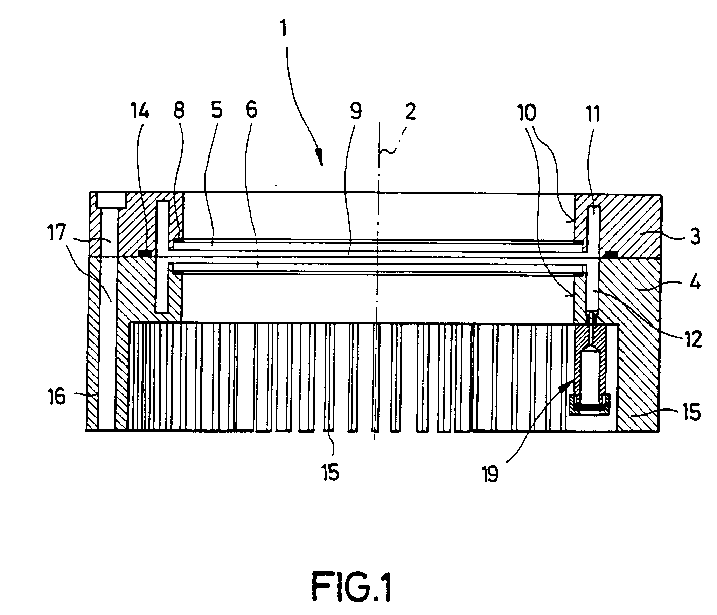

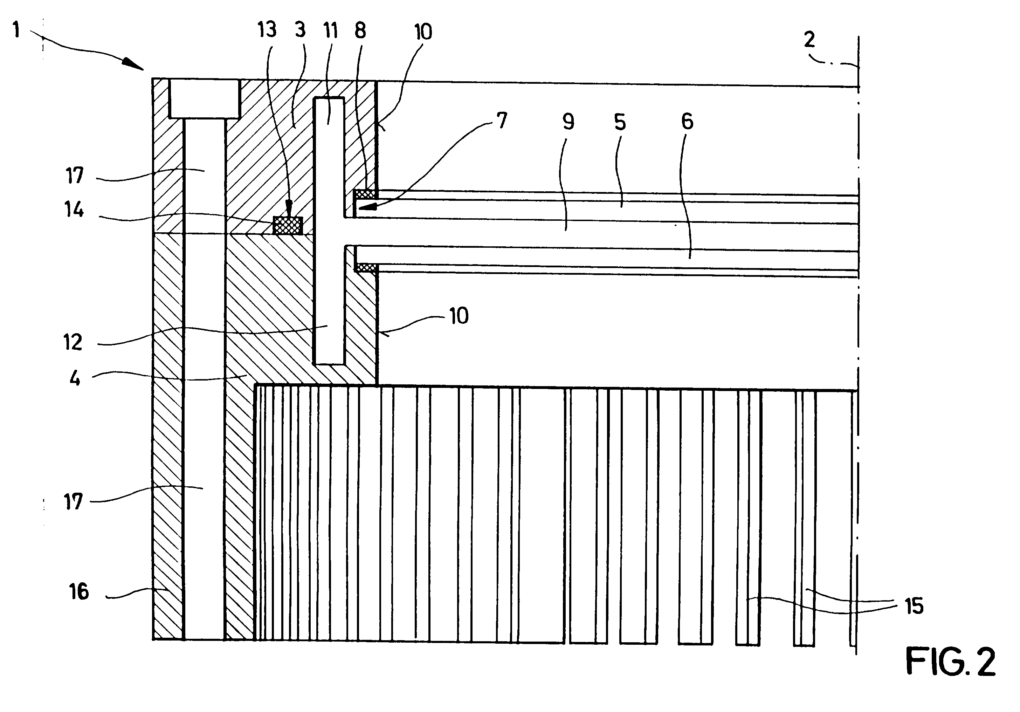

Die Fig. 1 und 2 zeigen einen Filter 1, der im Strahlengang einer nicht dargestellten Strahlenquelle einer Bestrahlungsvorrichtung angeordnet werden kann.

Der Filter 1 besteht im wesentlichen aus einem quer zur Filterachse zweigeteilten Rahmen, der aus einer ersten Rahmenhälfte 3 und einer zweiten Rahmenhälfte 4, sowie aus einer der Strahlenquelle abgewandten Filterscheibe 5 und einer dazu planparallel angeordneten zweiten Filterscheibe 6 besteht. Hierzu sind die ebenen Filterscheiben 5, 6 entsprechend in einer umlaufenden Stufe 7 der ersten und zweiten Rahmenhälfte 3, 4 paßgenau eingesetzt. Dabei liegen die Filterscheiben 5, 6 jeweils an ihrem Rand auf der dem Grund der Stufe 7 zugewandten Seite an einer umlaufenden Dichtung 8 an. Die Dichtung 8 kann z.B. aus einer geeigneten Silikonklebschicht bestehen, so daß die Filterscheiben 5, 6 gleichzeitig durch die Dichtung 8 in den entsprechenden Rahmenhälften 3, 4 befestigt sind. Die Stufe 7 ist jeweils so in den Rahmenhälften 3, 4 angeordnet, daß die beiden Filterscheiben 5, 6 mit einem bestimmten axialen Abstand zueinander angeordnet sind und somit einen dazwischenliegenden, scheibenförmigen Hohlraum 9 bilden. Dieser Hohlraum 9 ist mit einem das Strahlenspektrum selektiv beeinflußenden Medium gefüllt, um bestimmte Banden der von der Strahlenquelle abgegebenen Strahlung herauszufiltern. Üblicherweise besteht dieses Medium bei Filtern zum Einsatz zur Wärmetherapie aus Wasser, dem eventuell noch Fungizide beigesetzt sind.

Um diesen Strahlendurchtritt durch die beiden Filterscheiben 5, 6 zu gewährleisten, sind die beiden Rahmenhälften 3, 4 jeweils mit einer relativ großen, zur Filterachse 2 konzentrischen Durchgangsbohrung 10 versehen.

In den beiden Rahmenhälften 3, 4 sind jeweils konzentrisch zur Filterachse 2 angeordnete Ringkammerhälften 11 und 12 angeordnet, die mit dem Hohlraum 9 in Verbindung stehen. Hierzu ist der Hohlraum 9 lediglich entlang seines äußeren Randes gegenüber den Rindkammerhälften 11, 12 offen gelassen. Die Ringkammerhälften 11, 12 erstrecken sich jeweils parallel zur Filterachse 2 weitestmöglich in die jeweiligen Rahmenhälften 4, 5 hinein. Bevorzugt werden die Ringkammerhälften 11, 12 in Form von ringförmigen Einstichen in die Rahmenhälften 3, 4 eingebracht. Die Ringkammerhälften 11, 12 sind ebenfalls mit dem Medium gefüllt.

Damit auch die Ringkammerhälften 11, 12 nach außen hin abgedichtet sind, weist die erste Rahmenhälfte 3 eine umlaufende, gegenüber der Filterachse 2 außerhalb der Ringkammerhälften 11, 12 angeordnete Ringnut 13 auf, in die ein Dichtungsring 14 eingesetzt ist, der in der Teilungsebene der ersten und zweiten Rahmenhälfte 3, 4 mit der zweiten Rahmenhälfte 4 dichtend zur Anlage kommt.

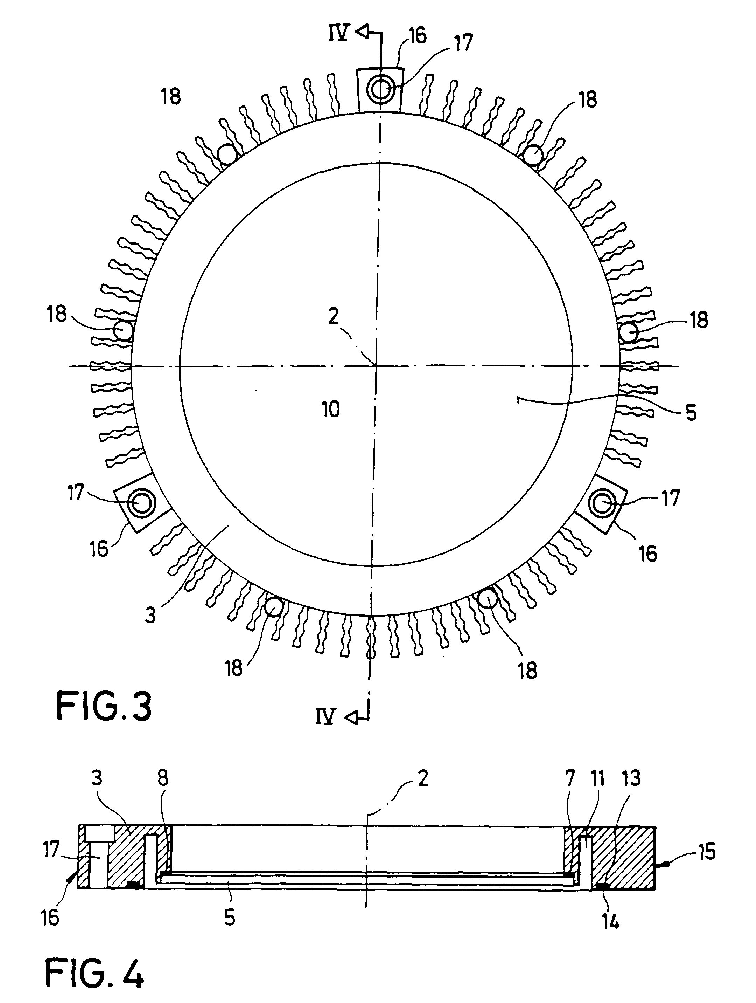

Wie insbesondere auch in den Darstellungen der einzelnen Rahmenhälften 3, 4 (siehe Figuren 3 bis 6) ersichtlich ist, sind am Umfang der ersten und zweiten Rahmenhälfte 3, 4 radial nach außen abstehende Kühlrippen 15 angeordnet, die zur Vergrößerung der Oberfläche und somit der Kühlwirkung leicht gewellt ausgeführt sein können. Die Kühlrippen 15 der zweiten Rahmenhälfte 4 sind wesentlich länger ausgebildet als die Kühlrippen 15 der ersten Rahmenhälfte 3 und sind auf einem Großteil ihrer Länge nicht mehr mit dem Hauptkörper der zweiten Rahmenhälfte 4 verbunden.

In regelmäßigen Abständen sind anstatt der relativ dünnen Kühlrippen vergrößerte Stege 16 am äußeren Umfang der Rahmenhälften 3, 4 in gleicher Längserstreckung wie die Kühlrippen 15 angeordnet. Die Stege 16 dienen zur Aufnahme von Längsbohrungen 17, in die nicht dargestellte Befestigungsmittel zur Befestigung am nicht dargestellten Gehäuse der Bestrahlungsvorrichtung einsetzbar sind. Des weiteren sind zwischen einigen Kühlrippen in regelmäßigen Abständen Materialanhäufungen 18 angeformt, die an der ersten und zweiten Rahmenhälfte 3, 4 fluchtende Gewindebohrungen aufweisen und in die ebenfalls nicht dargestellte Befestigungsmittel z.B. Schrauben zum Verbinden der beiden Rahmenhälften 3, 4 eingeführt werden können.

Die erste Filterscheibe 5 erwärmt sich durch das sich im Hohlraum 9 befindliche Medium nicht so stark, weshalb auch ein weniger warmfester Werkstoff eingesetzt werden kann. Die erste Filterscheibe 5 besteht deshalb aus Kunststoff, vorzugsweise Polycarbonat. Die zweite Filterscheibe 6 hingegen ist der Strahlenquelle direkt ausgesetzt und erhitzt sich deshalb beträchtlich, weshalb diese Scheibe bevorzugt aus einem hitzebeständigen Material, vorzugsweise Mineralglas, besteht.

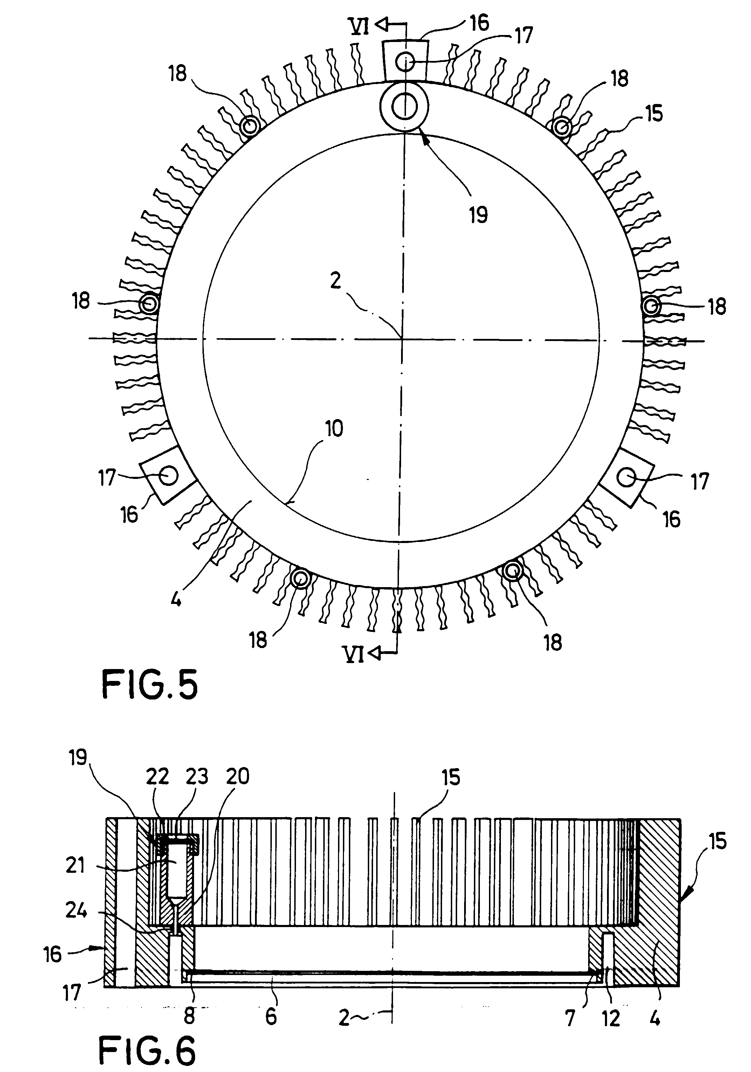

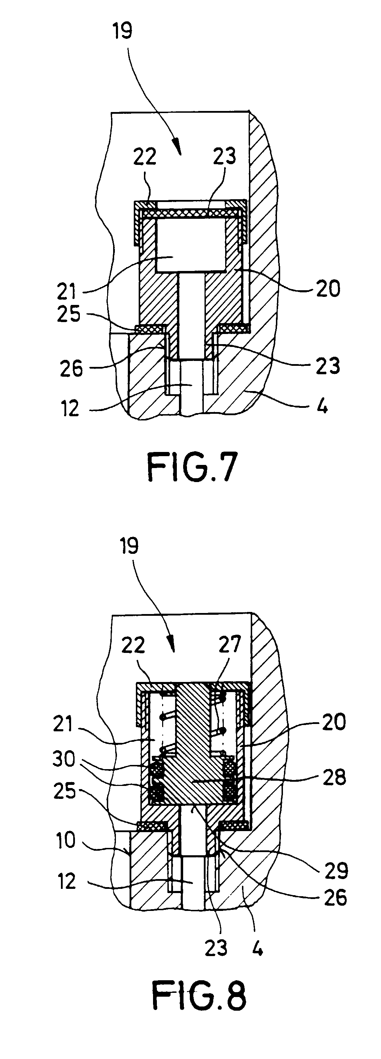

Wie insbesondere in Fig. 6 zu sehen ist, weist die zweite Rahmenhälfte 4 eine mit der Ringkammerhälfte 12 in Verbindung stehende Druckausgleichsvorrichtung 19 auf, die Druckschwankungen aufgrund der Wärmeausdehnung des Mediums im Hohlraum 9 ausgleicht, sowie eine eventuelle Ansammlung von Gasblasen aufnimmt. Die gezeigte Druckausgleichsvorrichtung 19 besteht aus einem auf die zweite Rahmenhälfte 4 aufgesetzten Ausgleichsbehälter 20, der eine Ausgleichskammer 21 aufweist, die mit einem Schraubdeckel 22 verschlossen ist. Der Schraubdeckel 22 weist ein Sichtfenster auf, unterhalb dessen eine flexible Membran 23 angeordnet ist. Am unteren Ende des Ausgleichsbehälters 20 befindet sich ein zylindrischer Hohlzapfen 24, der in eine Bohrung der zweiten Rahmenhälfte 4 eingepreßt oder, wie in Fig. 7 gezeigt, eingeschraubt ist und sich bis in die Ringkammerhälfte 12 erstreckt, um eine Verbindung zwischen Ringkammer 12 und Ausgleichskammer 21 herzustellen. Wichtig hierbei ist, daß die Verbindungsstelle zwischen Druckausgleichsvorrichtung 19 und zweite Rahmenhälfte 4 ebenfalls dicht ist. Die Variante einer Druckausgleichsvorrichtung gemäß Fig. 7 weist hierzu eine zwischen dem Ausgleichsbehälter 20 und der zweiten Rahmenhälfte 4 eine Dichtung 25 auf, die den mit einem Außengewinde 26 versehenen Hohlzapfen 23 umgibt.

In Fig. 8 ist eine weitere Variante einer Druckausgleichsvorrichtung dargestellt, bei der in der Ausgleichskammer 21 des Ausgleichsbehälters 20 ein von einer Druckfeder 27 einseitig belasteter Kolben 28 angeordnet ist. Damit das Medium sich nur innerhalb des von der Stirnseite 29 des Kolbens 28 einseitig begrenzten Bereich der Ausgleichskammer 21 befindet, weist der Kolben 28 an seinem Umfang mit der Ausgleichskammerwand in Anlage stehende Dichtungsringe 30 auf.

Um einen besseren Wärmeaustausch zu gewährleisten, sind die Rahmenhälften 3, 4 jeweils aus einem gut wärmeleitenden Material, wie z.B. Aluminium, hergestellt.

Im folgenden wird die Wirkungs- und Funktionsweise der vorliegenden Erfindung näher erläutert.

Zunächst wird die Strahlenquelle der Bestrahlungsvorrichtung eingeschaltet, so daß diese Strahlen in Richtung des Filters 1 aussendet. Diese Strahlen treffen zunächst auf die hitzebeständige zweite Filterscheibe 6 und dann auf das sich im Hohlraum 9 befindliche Medium, sowie anschließend auf die erste Filterscheibe 5. Durch die Erwärmung der zweiten Filterscheibe 6 erfolgt ebenfalls eine Erwärmung des Mediums im Hohlraum 9. Da die Ringkammerhälften 11, 12 mit dem Hohlraum 9 in Verbindung stehen und aufgrund ihrer relativ großen Oberfläche für eine gute Wärmeabführung von dem Medium an die Rahmenhälften 3, 4 sorgen, stellt sich eine natürliche Konvektion innerhalb des Mediums ein. Dieser Wärmeaustausch ist so gewählt, daß das Medium seinen Siedepunkt nicht erreicht. Aus diesem Grund und aufgrund, daß als Medium bevorzugt Wasser verwendet wird, ist die Erwärmung der ersten Filterscheibe 5 in bezug auf die zweite Filterscheibe relativ gering.

Neben der Möglichkeit aufgrund der vorliegenden Erfindung, die Ringkammerhälften 11, 12 in der oben beschriebenen Weise anzuordnen, um eine vergrößerte Oberfläche bereitzustellen, bietet die vorliegende Erfindung den Vorteil, daß aufgrund der Druckerhöhung infolge der Erwärmung des Mediums im Hohlraum 9 die Filterscheiben 5 und 6 auf ihre jeweiligen Dichtungen 8 gepreßt werden. Dies geschieht deshalb, weil die erste Filterscheibe 5 auf der dem Hohlraum 9 zugewandten Seite an der ersten Rahmenhälfte 3 und die zweite Filterscheibe 6 auf der dem Hohlraum 9 zugewandten Seite an der zweiten Rahmenhälfte 4 angeordnet ist. Somit trägt eine Druckerhöhung im Hohlraum 9 automatisch zu einer verbesserten Abdichtung bei. Der Druck darf jedoch nicht über einen bestimmten Wert schreiten, bei dem die Filterscheiben 5, 6 unzulässigerweise verformt werden und sich eine Linsenwirkung einstellen würde. Deshalb ist in Verbindung mit einer Ringkammerhälfte 12 eine Druckausgleichsvorrichtung 19 vorgesehen, deren flexible Membran 23 bei Druckerhöhung eher nachgibt, als daß sich die Filterscheiben 5, 6 unzulässig verformen. Des weiteren dient die Druckausgleichsvorrichtung 19 zur Aufnahme von etwaigen Gasblasen, die z.B. durch Diffusion von Gas entlang der äußeren Dichtung in die Kammer eingedrungen sind, aufzunehmen.

Bei der in Fig. 8 gezeigten Variante der Druckausgleichsvorrichtung drückt das sich bei Erwärmung ausdehnende Medium gegen den Kolben 28, wobei die Federkraft der Druckfeder 27 so gewählt ist, daß sich die Filterscheiben 5, 6 nicht unzulässigerweise durch Druckerhöhung im Hohlraum 9 verformen. Möglich ist es aber auch, anstelle einer Druckausgleichsvorrichtung einen einfachen abgedichteten Stopfen in die dafür vorgesehene Aufnahmebohrung einzusetzen oder einzuschrauben.

## Patentansprüche

1. Bestrahlungsvorrichtung mit einer Strahlenquelle und einem im Strahlengang angeordneten Filter (1), der zwei transparente, im wesentlichen planparallel zueinander angeordnete Filterscheiben (5,6) aufweist, die mit ihren umlaufenden Rändern in einem Rahmen aus gut wärmeleitendem Material gehalten sind, wobei die Filterscheiben (5,6) und der Rahmen einen geschlossenen Hohlraum (9) begrenzen, in dem ein das Strahlenspektrum selektiv beeinflussendes Medium vorgesehen ist, **dadurch gekennzeichnet,** daß der Rahmen im wesentlichen quer zur Filterachse (2) in eine erste und eine zweite Rahmenhälfte (3,4) derart geteilt ist, daß die eine Filterscheibe (5) auf der dem Hohlraum (9) zugewandten Seite an der ersten Rahmenhälfte (3) angeordnet ist und die andere Filterscheibe (6) auf der dem Hohlraum (9) zugewandten Seite an der zweiten Rahmenhälfte (4) angeordnet ist.

2. Bestrahlungsvorrichtung nach Anspruch 1, **dadurch gekennzeichnet,** daß zumindest eine der Filterscheiben (5,6) entsprechend in einer umlaufenden Stufe (7) der ersten und/oder zweiten Rahmenhälfte (3,4) im wesentlichen paßgenau eingesetzt ist.

3. Bestrahlungsvorrichtung nach Anspruch 1 oder 2, **dadurch gekennzeichnet,** daß die Filterscheiben (5,6) jeweils an ihrem Rand auf der dem Hohlraum (9) abgewandten Seite an einer im wesentlichen umlaufenden Dichtung (8) anliegen.

4. Bestrahlungsvorrichtung nach einem der Ansprüche 1 bis 3, **dadurch gekennzeichnet,** daß mindestens in einer der Rahmenhälften (3,4) eine umlaufende, mit dem Hohlraum (9) in Verbindung stehende Ringkammer (11,12) angeordnet ist.

5. Bestrahlungsvorrichtung nach Anspruch 4, **dadurch gekennzeichnet,** daß in beiden Rahmenhälften (3,4) jeweils eine Ringkammerhälfte (11,12) zum Bilden einer gemeinsamen großen Ringkammer angeordnet ist.

6. Bestrahlungsvorrichtung nach einem der Ansprüche 4 und 5, **dadurch gekennzeichnet,** daß außerhalb der Ringkammer mindestens eine umlaufende Dichtung (14) in einer Rahmenhälfte (3,4) angeordnet ist, die an der anderen Rahmenhälfte (3,4) anliegt.

7. Bestrahlungsvorrichtung nach einem der Ansprüche 4 bis 6, **dadurch gekennzeichnet,** daß in oder an der Ringkammer eine Druckausgleichsvorrichtung (19) vorgesehen ist, die Druckschwankungen aufgrund des Erwärmens des im Hohlraum (9) befindlichen Mediums ausgleicht, sowie eine eventuelle Ansammlung von Gasblasen aufnimmt.

8. Bestrahlungsvorrichtung nach Anspruch 7, **dadurch gekennzeichnet,** daß die Druckausgleichsvorrichtung (19) aus einem mit der Ringkammer in Verbindung stehenden Ausgleichsbehälter (20) besteht, der eine Ausgleichskammer (21) aufweist, die zumindest einseitig von einer flexiblen Membran (23) begrenzt ist.

9. Bestrahlungsvorrichtung nach Anspruch 7, **dadurch gekennzeichnet,** daß die Druckausgleichsvorrichtung (19) aus einem mit der Ringkammer in Verbindung stehenden Ausgleichsbehälter (20) besteht, der eine Ausgleichskammer (21) aufweist, in der ein federbelasteter Kolben angeordnet ist, der bei Druckanstieg im Hohlraum (9) entsprechend einfedert.

10. Bestrahlungsvorrichtung nach einem der Ansprüche 1 bis 9, **dadurch gekennzeichnet,** daß die der Strahlenquelle abgewandte Filterscheibe (5) des Filters (1) aus Kunststoff, vorzugsweise Polycarbonat, besteht.

11. Bestrahlungsvorrichtung nach einem der Ansprüche 1 bis 10, **dadurch gekennzeichnet,** daß die der Strahlenquelle zugewandte Filterscheibe (6) aus einem hitzebeständigen Material, vorzugsweise Mineralglas, besteht.

12. Bestrahlungsvorrichtung nach einem der Ansprüche 1 bis 11, **dadurch gekennzeichnet,** daß der Rahmen an seinem Außenumfang Kühlrippen (15) aufweist.

13. Bestrahlungsvorrichtung nach Anspruch 12, **dadurch gekennzeichnet,** daß einige Kühlrippen (15) als verdickte Stege (16) ausgebildet sind und entsprechend bezüglich der ersten und zweiten Rahmenhälfte (3,4) fluchtende Durchgangsbohrungen (17) zur Aufnahme von Befestigungsmitteln aufweisen.

## Claims

1. A radiation apparatus comprising a radiation source and a filter (1) which is arranged in an optical path and has two transparent filter discs (5, 6) which are substantially arranged in plane-parallel configuration relative to each other and which are held with their surrounding edges in a frame made of a material of good heat conduction, said filter discs (5, 6) and said frame defining a closed cavity (9) which has provided therein a medium for selectively influencing the radiation spectrum, **characterized in** that said frame is divided, substantially in a direction transverse to filter axis (2), into first and second frame halves (3, 4) in such a manner that said one filter disc (5) is arranged on said first frame half (3) at the side facing said cavity (9) and said other filter disc (6) is arranged on said second frame half (4) at the side facing said cavity (9).

2. The radiation apparatus according to claim 1,
**characterized in** that at least one of said filter discs (5, 6) is inserted substantially with a snug fit in a surrounding step (7) of said first and/or second frame halves (3, 4).

3. The radiation apparatus according to claim 1 or 2,
**characterized in** that on their edges said filter discs (5, 6) rest each on a substantially surrounding seal (8) at the side facing away from said cavity (9).

4. The radiation apparatus according to any one of claims 1 to 3, **characterized in** that a surrounding annular chamber (11, 12) which communicates with said cavity (9) is arranged at least in one of said frame halves (3, 4).

5. The radiation apparatus according to claim 4,
**characterized in** that said two frame halves (3, 4) have each arranged therein an annular chamber half (11, 12) for forming a common and large annular chamber.

6. The radiation apparatus according to any one of claims 4 and 5, **characterized in** that at least one surrounding seal (14) is arranged outside of said annular chamber in a frame half (23,4) which rests on the other frame half (3, 4).

7. The radiation apparatus according to any one of claims 4 to 6, **characterized in** that said annular chamber has provided therein or thereon a pressure compensating device (19) which compensates pressure variations caused by the heating of the medium within said cavity (9), and receives a possible accumulation of gas bubbles.

8. The radiation apparatus according to claim 7,
**characterized in** that said pressure compensating device (19) consists of a compensating container (20) which is in communication with said annular chamber and comprises a compensating chamber (21) which is defined at least at one side by a flexible membrane (23).

9. The radiation apparatus according to claim 7,
**characterized in** that said pressure compensating device (19) consists of a compensating container (20) which is in communication with said annular chamber and comprises a compensating chamber (21) having arranged therein a spring-loaded piston which resiliently moves in upon increase in pressure in said cavity (9).

10. The radiation apparatus according to any one of claims 1 to 9, **characterized in** that the filter disc (5) of said filter (1) which faces away from said radiation source consists of a plastic material, preferably polycarbonate.

11. The radiation apparatus according to any one of claims 1 to 10, **characterized in** that said filter disc (6) which faces said radiation source consists of a heat-resistant material, preferably mineral glass.

12. The radiation apparatus according to any one of claims 1 to 11, **characterized in** that said frame has cooling ribs (15) on its circumference.

13. The radiation apparatus according to claim 12,
**characterized in** that some cooling ribs (15) are formed as thickened webs (16) and comprise through-holes (17) aligned with respect to said first and second frame halves (3, 4) for receiving fastening means.

## Revendications

1. Dispositif d'irradiation comprenant une source de rayonnement et un filtre (1) monté sur la trajectoire des rayons, lequel filtre est muni de deux disques de filtrage (5, 6) transparents, sensiblement plans et parallèles l'un à l'autre, dont les bords périphériques sont maintenus dans un cadre réalisé en un matériau bon conducteur thermique, les disques de filtrage (5, 6) et le cadre délimitant une cavité (9) fermée, dans laquelle est prévu un milieu influençant de manière sélective le spectre de rayonnement, caractérisé en ce que le cadre est partagé, sensiblement dans le sens transversal à l'axe du filtre (2), en une première et une deuxième moitié de cadre (3, 4), de telle sorte que l'un des disques de filtrage (5) est monté contre la première moitié de cadre (3) sur le côté orienté vers la cavité (9) et l'autre disque de filtrage (6) est monté contre la deuxième moitié de cadre (4) sur le côté orienté vers la cavité (9).

2. Dispositif d'irradiation selon la revendication 1, caractérisé en ce qu'au moins l'un des disques de filtrage (5, 6) est inséré, sensiblement de manière ajustée, dans un décrochement périphérique (7) de la première et/ou de la deuxième moitié de cadre (3, 4).

3. Dispositif d'irradiation selon la revendication 1 ou 2, caractérisé en ce que les disques de filtrage (5, 6) viennent chacun en contact par leur bord contre un joint d'étanchéité (8) sensiblement concentrique posé contre le côté opposé à la cavité (9).

4. Dispositif d'irradiation selon l'une quelconque des revendications 1 à 3, caractérisé en ce qu'au moins dans l'une des moitiés de cadre (3, 4) est disposée une chambre annulaire (11, 12) concentrique qui communique avec la cavité (9).

5. Dispositif d'irradiation selon la revendication 4, caractérisé en ce qu'une moitié de chambre annulaire (11, 12) est disposée dans chacune des deux moitiés de cadre (3, 4) pour former une grande chambre annulaire commune.

6. Dispositif d'irradiation selon la revendication 4 ou 5, caractérisé en ce que, en dehors de la chambre annulaire, au moins un joint d'étanchéité (14) concentrique est posé dans une moitié de cadre (3, 4), qui vient en appui contre l'autre moitié de cadre (3, 4).

7. Dispositif d'irradiation selon l'une quelconque des revendications 4 à 6, caractérisé en ce qu'il est prévu, dans ou contre la chambre annulaire, un dispositif d'égalisation de pression (19), qui équilibre les variations de pression dues à l'échauffement du milieu contenu dans la cavité (9), et qui absorbe une éventuelle accumulation de bulles de gaz.

8. Dispositif d'irradiation selon la revendication 7, caractérisé en ce que le dispositif d'égalisation de pression (19) est formé par un réservoir d'égalisation (20) qui communique avec la chambre annulaire, lequel réservoir d'égalisation comporte une chambre d'égalisation (21) qui est délimitée au moins sur un côté par une membrane (23) flexible.

9. Dispositif d'irradiation selon la revendication 7, caractérisé en ce que le dispositif d'égalisation de pression (19) est formé par un réservoir d'égalisation (20) qui communique avec la chambre annulaire et qui comporte une chambre d'égalisation (21) dans laquelle est monté un piston sollicité par un ressort, qui se comprime de manière correspondante à chaque augmentation de la pression dans la cavité (9).

10. Dispositif d'irradiation selon l'une quelconque des revendications 1 à 9, caractérisé en ce que le disque de filtrage (5) du filtre (1), opposé à la source de rayonnement, est réalisé en matière plastique, de préférence en polycarbonate.

11. Dispositif d'irradiation selon l'une quelconque des revendications 1 à 10, caractérisé en ce que le disque de filtrage (6), orienté vers la source de rayonnement, est réalisé en un matériau résistant aux températures élevées, de préférence en verre minéral.

12. Dispositif d'irradiation selon l'une quelconque des revendications 1 à 11, caractérisé en ce que le bord extérieur du cadre comporte des ailettes de refroidissement (15).

13. Dispositif d'irradiation selon la revendication 12, caractérisé en ce que quelques ailettes de refroidissement (15) sont conçues en forme de traverses (16) plus épaisses et sont munies de trous débouchants (17) alignés de manière correspondante par rapport à la première et à la deuxième moitié de cadre (3, 4) et destinés à recevoir des moyens de fixation.
